# EUROPÄISCHE PATENTSCHRIFT

(11) **EP 0 425 887 B1**
(45) Veröffentlichungstag und Bekanntmachung des Hinweises auf die Patenterteilung: **23.03.1994**
(21) Anmeldenummer: 90119820.0
(22) Anmeldetag: 16.10.1990
(51) Int. Cl.: C07C 17/00, B01J 23/18, B01J 23/10

(54) **Verfahren zum Halogen-Fluor-Austausch in organischen Verbindungen und Katalysatoren hierzu**
Process for halogen-fluor exchange in organic compounds and catalysts therefor
Procédé pour l'échange halogène-fluor dans de composés organiques et catalyseurs utilisables dans ces procédés

(30) Priorität: 28.10.1989 DE 3936024
(43) Veröffentlichungstag der Anmeldung: 08.05.1991
(73) Patentinhaber: BAYER AG, 51368 Leverkusen (DE)
(72) Erfinder: Schwarz, Hans-Helmut, Dr., W-4150 Krefeld (DE); Braden, Rudolf, Dr., W-5068 Odenthal (DE)

(56) Entgegenhaltungen:
- EP-A- 0 300 724
- EP-A- 0 301 346
- DE-A- 1 900 241

## Beschreibung

Die vorliegende Erfindung betrifft ein Verfahren zum Austausch von Halogen, das verschieden von Fluor ist, bevorzugt von Chlor, gegen Fluor in organischen Verbindungen mittels Fluorwasserstoff. Die Erfindung betrifft weiterhin Wismut/Erdalkalimetall-Katalysatoren für dieses Verfahren, die 0,005 - 0,8 gA Wismut pro Mol der eingesetzten Erdalkaliverbindung enthalten und gegebenenfalls Promotoren und/oder inerte Zusatzstoffe.

Für den Austausch von Halogen, das verschieden von Fluor ist (hauptsächlich Chlor), gegen Fluor in halogenierten organischen Verbindungen mittels Fluorwasserstoff, wobei hauptsächlich in der Gasphase gearbeitet wird, ist eine Reihe von Katalysatoren vorgeschlagen worden, wie Chrom und Magnesium enthaltende Katalysatoren (EP-130 532), Magnesium und Eisen, sowie zusätzlich Kupfer und/oder Mangan enthaltende Katalysatoren (EP-235 547), Chromoxidgele (US-3 149 170); bekannt sind weiter Katalysatoren für die Hydrofluorierung von Alkenen oder Fluoralkenen zu den zugehorigen fluorierten Alkanen, die aus Aluminiumoxid bestehen, welches Halogenide von Chrom, Kobalt, Nickel, Kupfer, Palladium oder Wismut enthält (DE-AS 19 00 241). Unter den genannten Katalysatoren besitzen besonders die Chromoxidgele eine hohe Aktivität. Ihre Herstellung und ihr Einsatz ist jedoch mit fölgenden Nachteilen behaftet: Bei der Herstellung dieser Gele aus wäßriger Lösung muß mit erheblichen Filtrationsschwierigkeiten gerechnet werden; von besonderer und nachteiliger Bedeutung sind jedoch die Umweltschutzmaßnahmen, die ergriffen werden müssen, um chromhaltige Reste aus der Herstellung dieser Katalysatoren oder verbrauchte chromhaltige Katalysatoren selbst zu entsorgen.

Es bestand daher ein Bedürfnis nach Verfahren zum Halogen-Fluor-Austausch in organischen Verbindungen mit Fluorwasserstoff, insbesondere in der Gasphase, die mit hoher Fluorierungsaktivität und Fluorierungsselektivität durchführbar sind und die mit Katalysatoren arbeiten, die chromfrei sind. Das erfindungsgemäße Verfahren und die erfindungsgemäßen Katalysatoren erfüllen dieses Bedürfnis,

Es wurde ein Verfahren zum Austausch von Halogen, das verschieden von Fluor ist, besonders von Chlor, gegen Fluor in organischen Verbindungen mittels Fluorwasserstoff in Gegenwart eines Katalysators gefunden, das dadurch gekennzeichnet ist, daß ein chromfreier Wismut/ Erdalkali-Katalysator eingesetzt wird, der 0,005-0,8 gA Wismut, bevorzugt 0,008-0,7 gA Wismut, pro Mol der eingesetzten Erdalkaliverbindung enthält und gegebenenfalls als Promotoren 0,01 bis 200 g-Atome (bezogen auf Wismut) mindestens einer Verbindung von Elementen der VII. und/oder VIII. Nebengruppe des Periodensystems der Elemente (Mendelejew) und/oder Lanthanide und/oder der inerte Zusatzstoffe enthält, man in Gegenwart von 0,1 bis 1,5 Mol Chlor in elementarer Form pro Mol der umzusetzenden organischen Verbindung arbeitet und die Katalysatoren bei 20 bis 500°C mit überschüssigem Fluorwasserstoff vorbehandelt.

Das erfindungsgemäße Verfahren ist demnach vor allem gekennzeichnet durch den Einsatz eines der oben genannten Katalysatoren. Selbstverständlich können auch mehrere der genannten Katalysatoren eingesetzt werden. Alle erfindungsgemäß einsetzbaren Katalysatoren zeichnen sich durch ihre Chromfreiheit aus.

Die Erfindung betrifft daher auch die oben genannten Wismut-Katalysatoren für dieses Verfahren, die gegebenenfalls Promotoren und/oder inerte Zusatzstoffe enthalten können.

Die erfindungsgemäßen Katalysatoren enthalten neben Wismut mindestens eine Verbindung von Erdalkalimetallen.

In bevorzugter Weise handelt es sich bei den zur Herstellung der oben genannten Katalysatoren eingesetzten Erdalkalimetallverbindungen um die Carbonate, Hydroxide, Oxide und Fluoride; in besonders bevorzugter Weise wird mindestens eine Verbindung aus der Gruppe von Magnesiumoxid, Calciumoxid und Bariumoxid eingesetzt. Die eingesetzten Erdalkaliverbindungen werden mit einer oder mehrerer Wismutverbindungen beladen oder getränkt, wobei auch eine doppelte Umsetzung zwischen der Erdalkaliverbindung und der (den) Wismutverbindung(en) eintreten kann.

Beispiele für Promotoren sind Verbindungen von Mangan, Eisen, Kobalt, Nickel, Lanthan, Cer und Dysprosium. Die Katalysatoren können auch mehr als einen Promotor tragen. Das Grammatomverhältnis der Promotorelemente zum Element Wismut beträgt in bevorzugter Weise 0,1-10:1.

Die erfindungsgemäßen Katalysatoren können fernerhin inerte Zusatzstoffe, beispielsweise Graphit, enthalten; in bevorzugter Weise sind sie jedoch frei von solchen inerten Zusatzstoffen.

Zur Herstellung der erfindungsgemäßen Katalysatoren können die Erdalkaliverbindungen in üblicher Weise mit Lösungen des Wismuts bzw. der Promotoren getränkt oder besprüht werden. Des weiteren können die Erdalkalimetallverbindungen mit löslichen oder unlöslichen Verbindungen des Wismuts bzw. der Promotoren und Wasser angeteigt werden, woraufhin aus der entstehenden teigigen Paste Katalysatorformlinge hergestellt und getrocknet werden, Für den Fall des Anteigens löslicher Salze des Wismuts bzw. der Promotorelemente und der Erdalkalimetallverbindungen tritt vielfach eine doppelte Umsetzung, beispielsweise zwischen Wismutnitrat und Calciumoxid ein. Die genannten Herstellungsverfahren sind dem Fachmann grundsätzlich bekannt.

Im vorliegenden Falle ist das Anteigen die eleganteste und vorteilhafteste Herstellungsmethode und soll daher im folgenden näher beschrieben werden. Hierzu werden die Carbonate, Hydroxide, Oxide oder Fluoride der Erdalkalimetalle beispielsweise mit wasserlöslichen Salzen des Wismuts und der Promotorelemente in wäßrigem Medium bei 5-95°C zur Einstellung des Reaktionsgleichgewichts gründlich durchmischt, Falls inerte Zusätze gewünscht sind, werden diese ebenfalls in dieser Phase zugesetzt. Zur besseren Handhabung der wäßrigen Lösung von Wismutsalzen und gegebenenfalls Promotormetallsalzen werden diese mit etwas Säure versetzt, um die Hydrolyse und damit ein Ausflocken basischer Salze zu vermeiden. Grundsätzlich ist jedoch auch der Einsatz von schwerlöslichen oder unlöslichen basischen Salzen des Wismuts oder der Promotormetalle möglich, hierbei ist die Einstellung des Reaktionsgleichgewichtes jedoch langsamer.

Für die Einstellung des Reaktionsgleichgewichtes sind 1-5 Stunden bei guter Durchmischung anzusetzen, da das Anteigen und eine hierbei erfolgende doppelte Umsetzung in nicht homogener Phase erfolgt. Weiterhin wird die Einstellung des Reaktionsgleichgewichtes bei erhöhter Temperatur bis zu 95°C schneller erreicht; im allgemeinen kann man jedoch bei 20-50°C, bevorzugt bei Raumtemperatur arbeiten.

Die zum Anteigen angewandte Wassermenge ist für eine etwa erfolgende doppelte Umsetzung nicht kritisch, sondern wird durch einfache Vorversuche so bestimmt, daß die sich bildende pastöse Masse durch einen Kneter verarbeitbar ist. Die Reihenfolge der Zugabe von Wasser, Erdalkalimetallverbindungen und Metallsalzen ist grundsatzlich beliebig.

Die erhaltene Paste wird ohne Waschen getrocknet. Sie ist vor oder nach dem Trocknen zur Herstellung von Katalysatorformkörpern geeignet. Da bei dieser Art der Katalysatorherstellung kein Auswaschvorgang eingeschaltet wird, ist das Atomverhältnis der Einsatzkomponenten dem des fertigen Katalysators gleich. Daneben entstehen keine entsorgungsbedürftigen Abwässer. Die Formkörper können mit den üblichen verfahrenstechnischen Maßnahmen, wie beispielsweise Pelletisieren, Extrudieren oder Granulieren hergestellt werden.

Nach der Formgebung werden die Katalysatorformlinge getrocknet, was zu mechanisch sehr stabilen Katalysatorformlingen führt. Die Trocknung wird bei 50-150°C, vorzugsweise 70-120°C durchgeführt; hierzu kann sowohl unter Normaldruck als auch unter Vakuum gearbeitet werden. Die Trocknung ist grundsätzlich auch bei Raumtemperatur möglich, benötigt dann aber eine längere Zeit.

Es wurde weiterhin festgestellt, daß die so erhaltenen Katalysatoren im Verlaufe ihres Einsatzes für die Fluorierung organischer Verbindungen mittels Fluorwasserstoff einen Aktivitätsanstieg erfahren. Daher werden die erfindungsgemäßen Katalysatoren vor ihrem Einsatz durch eine Behandlung mit überschüssigem Fluorwasserstoff zu aktiviert. Diese Behandlung mit Fluorwasserstoff wird bei 20-500°C, bevorzugt 100-500°C, besonders bevorzugt 120-420°C durchgeführt, Diese Behandlung führt zu einem weitgehenden oder vollständigen Ersatz vorliegender Anionen durch das Fluoridanion. Diese Behandlung der Katalysatoren mit Fluorwasserstoff kann vorteilhafterweise dadurch ergänzt werden, daß man nach dem Trocknen, jedoch vor der Behandlung mit Fluorwasserstoff, die Katalysatorformlinge bei 200-400°C, bevorzugt 300-375°C tempert. Während dieser Temperung werden zersetzliche Anionen, beispielsweise das Nitratanion, zerstört und damit die nachfolgende Umwandlung in das Fluoridanion erleichtert.

Man erhält bereits voll wirksame Katalysatoren, wenn pro Mol eingesetzter Metallverbindung 2 Mol Fluorwasserstoff zur Behandlung vorliegen. Auch höhere Fluorwasserstoffmengen sind möglich; ihr Einsatz ist nur durch wirtschaftliche Erwägungen begrenzt. Die Behandlungsdauer mit Fluorwasserstoff kann in weiten Grenzen gewählt werden; in bevorzugter Weise wird diese Zeit mit 0,5-10 Stunden angesetzt. Zur Vermeidung unerwünschter Temperaturspitzen kann man den Fluorwasserstoff durch Inertgas, wie Stickstoff oder Luft, verdünnen. Die mit der Inertgasverdünnung verbundene Vergrößerung des Gasvolumens ist weiterhin geeignet, entstehendes Wasser und andere flüchtige Behandlungsprodukte aufzunehmen. Für den Fall, daß eine solche Behandlung im Umluftverfahren erfolgt, werden diese flüchtigen Behandlungsprodukte dem Katalysator rascher entzogen. In vorteilhafter Weise erfolgt eine solche Behandlung mit Fluorwasserstoff in der gleichen Apparatur, in der der Katalysator letztendlich eingesetzt wird.

Die so erhaltenen Katalysatoren sind von ihrer mechanischen Stabilität her geeignet zum Einsatz in Festbett-, Fließbett- und Wirbelschichtreaktoren.

Die erfindungsgemäßen Katalysatoren sind grundsätzlich auch ohne Promotorzusatz brauchbar; der Zusatz der Promotoren bewirkt aber in vielen Fällen einen weiteren deutlichen Anstieg der Aktivität und Selektivität. Inerte Zusatzstoffe können helfen, die Gesamtkinetik der Fluorierungsreaktion organischer Verbindungen zu beeinflussen, beispielsweise Diffusions-, Adsorptions- oder Desorptionsvorgänge der Reaktionspartner. Dies kann etwa durch den Zusatz poröser inerter Zusatzstoffe erfolgen. Inerte Zusatzstoffe können ferner geeignet sein, einen Verdünnnngseffekt der wirksamen Katalysatorbestandteile in den Katalysatorformlingen zu bewirken und so einen moderierenden Einfluß auf die Fluorierungsreaktion zu bewirken. Die essentiellen Metallanteile sind in jedem Fall in der Wirksubstanz des Katalysators gleichmäßig statistisch verteilt. Für die meisten Einsatzzwecke ist die Verwendung eines von inerten Zusatzstoffen freien erfindungsgemäßen Katalysators ausreichend und daher bevorzugt.

Das Verfahren zum Austausch von Halogen, das verschieden von Fluor ist, besonders von Chlor, gegen Fluor wird vorzugsweise in der Gasphase bei einer Temperatur von 100-500°C durchgeführt,

Organische Verbindungen, die einer solchen Reaktion unterworfen werden können, sind beispielsweise halogenierte, bevorzugt chlorierte Kohlenwasserstoffe, halogenierte (chlorierte) Ketone, halogenierte (chlorierte) Ether, halogenierte (chlorierte) cyclische Acetale, Carbonsäure(ortho)-halogenide(-chloride), halogenierte (chlorierte) Nitrile, Säurehalogenide (-chloride), chlorierte Heterocyclen und andere. Besonders wichtig ist das erfindungsgemäße Verfahren für den Halogen(Chlor)-Fluor-Austausch in halogenierten (chlorierten) Kohlenwasserstoffen, beispielsweise in perchlorierten geradkettigen oder verzweigten Alkanen, Alkenen oder Alkadienen mit 2-8 C-Atomen oder in perchlorierten Cycloalkenen oder Cycloalkadienen mit 4-7 C-Atomen. Hervorgehoben sei die Herstellung von Tetrafluordichlor-cyclobuten aus Hexachlor-butadien und HF im erfindungsgemäßen Verfahren.

Ein weiteres Merkmal der vorliegenden Erfindung ist, daß neben Fluorwasserstoff auch elementares Chlor im Fluorierungsgemisch in einer Menge von 0,1-1,5 Mol, bevorzugt 0,2-1,1 Mol, pro Mol der umzusetzenden organischen Verbindung vorhanden ist.

Bei umzusetzenden offenkettigen oder cyclichen perhalogenierten C₄-Alkenen oder -Alkadienen wurde beobachtet, daß der Zusatz von elementarem Chlor die Bildung der offenkettigen C₄-Struktur begünstigt, Diese Begünstigung wird mit zunehmender Menge des elementaren Chlors stärker und ist im unteren Teil des angegebenen Bereiches nur schwach ausgeprägt. Dies stellt eine geeignete Maßnahme zur Steuerung des Produktspektrums dar.

### Beispiel 1

2,5 Gew.-Teile Bi(NO₃)₃.5 H₂O und 1,2 Teile 65 %ige Salpetersäure wurden in 3,6 Teilen Wasser gelöst. Dazu gab man 1 Teil Fe(NO₃)₃.9 H₂O. Diese Lösung wurde zu 1,5 Gew.-Teilen Magnesiumoxid gegeben und die dabei entstehende pastöse Masse innig verknetet. Anschließend wurde das pastöse Reaktionsprodukt granuliert und 16 h bei 100°C getrocknet. Anschließend wurde der Katalysator 6 h auf 400°C erhitzt. Das Atomverhältnis Mg:Bi:Fe betrug 1:1,29:0,17.

0,33 l der Kontaktkörper wurden in einem Rohr mit 5 cm lichter Weite und 100 cm Länge bei 350°C mit 5 Mol Fluorwasserstoff behandelt. Die Dauer der Fluorwasserstoffbehandlung betrug ca. 3 h. Dabei wurde HF mit N₂ im Molverhältnis 1:2 verdünnt.

Über 200 ml dieses Katalysators wurden im Laufe von 5 h 102 g Hexachlorcyclopentadien, 210 g HF und 3 l Chlor bei 350°C geleitet. Die Reaktionsgase wurden in einem Eis-Wasser-Gemisch kondensiert. Es schieden sich 72,4 g einer organischen Phase ab, die nach gaschromatographischer (GC) Analyse 58,4 % 1,2-Dichlor-3,3,4,4,5,5-hexafluorcyclopenten, 29,1 % 1,2,4-Trichlor-3,3,4,5,5-pentafluorcyclopenten und 8,1 % 1,2,4,4-Tetrachlor-3,3,5,5-tetrafluorcyclopenten enthielten. Hexachlorcyclopentadien hatte sich vollkommen umgesetzt.

### Beispiel 2

110,4 g BiCl₃ wurden in 150 g 18 %iger Salzsäure gelöst. Hiermit vermischte man eine Lösung von 16,76 g FeCl₃.6 H ₂O in 30 g Wasser. In einem Kneter legte man 250 g MgO vor und gab allmählich die Wismut-Eisen-Salzlösung hinzu. Durch exotherme Reaktion verdampfte Wasser, das man durch 170 ml H₂O ersetzte, Nach 1,5-stündiger Knetzeit wurde die Masse getrocknet, gemahlen, mit 2 % Graphit versetzt und gepillt. Das Atomverhältnis Mg:Bi:Fe betrug 1:0,06:0,04. Der Katalysator wurde wie im Beispiel 1 mit HF vorbehandelt.

Über 200 ml dieses Katalysators leitete man im Laufe von 5 h bei 420°C 99,6 g Hexachlorbutadien, 120 g HF und 22 g Chlor. Die Reaktionsgase wurden wie im Beispiel 1 kondensiert. Es bildete sich eine organische Phase von 81,5 g, die nach GC-Analyse 73,6 % 2,3-Dichlor-1,1,1,4,4,4-hexafluorbuten-2, 5,7 % 2-Chlor-1,1,1,3,4,4,4-heptafluorbuten und nur noch 6,1 % Hexachlorbutadien enthielt.

### Beispiel 3

Nach der Arbeitsweise des Beispiels 2 wurde ein Katalysator aus 30 g 18 %iger Salzsäure, 20 g BiCl₃, 150 g Wasser, 94,6 g FeCl₃.6 H₂O und 250 g MgO hergestellt und aktiviert.

Das Atomverhältnis Mg:Bi:Fe betrug 1:0,01:0,06.

Bei 425°C wurden über 200 ml Katalysator 99,6 g Hexachlorbutadien, 110 g HF und 27 g Chlor geleitet. Nach Kondensation der Reaktionsgase lieferte eine GC-Analyse der 82,1 g schweren organischen Phase folgende Werte: 5,9 % 2-Chlor-1,1,1,3,4,4,4-heptafluorbuten, 11,3 % 2-Chlor-1,1,1,4,4,4-hexafluorbuten, 72,9 % 2,3-Dichlor-1,1,1,4,4,4-hexafluorbuten und 4,9 % Hexachlorbutadien.

### Beispiel 4

Nach der Arbeitsweise des Beispiels 2 wurde ein Katalysator aus 30 g 18 %iger Salzsäure, 20 g BiCl₃, 320 g Wasser, 16,76 g FeCl₃.6 H₂O und 250 g MgO hergestellt und aktiviert.

Das Atomverhältnis Mg:Bi:Fe im Katalysator betrug 1:0,01:0,01.

Über 220 ml Katalysator wurden im Laufe von 5 h bei 420°C 99,6 g Hexachlorbutadien, 127,2 g HF und 27 g Chlor geleitet. Bei der Absorption der Reaktionsgase in Eiswasser entstanden 77,03 g einer organischen Phase, die 69,3 % 2,3-Dichlor-1,1,1,4,4,4-hexafluorbuten-2, 5,6 % 2-Chlor-1,1,1,3,4,4,4-heptachlorbuten-2 und 9,2 % Hexachlorbutadien enthielt.

### Beispiel 5

Nach der Arbeitsweise des Beispiels 1 wurde ein Katalysator aus 295 g Wasser, 25 g 65 %iger Salpetersäure, 50 g Fe(NO₃)₃.9 H₂O, 340 g Bi(NO₃)₃.5 H₂O und 500 g Bariumoxid hergestellt und bei 370°C mit HF aktiviert.

Das Atomverhältnis Ba:Bi:Fe betrug 1:0,007:0,05.

102 g Hexachlorcyclopentadien, 125 g HF und 27 g Chlor wurden im Laufe von 5 h über 200 ml Katalysator bei 400°C geleitet. Es bildeten sich 95,5 g organische Phase, die 5,1 % 1,2,4-Trichlor-3,3,4,5,5-pentafluorcyclopenten-1, 64,8 % 1,2,4,4-Tetrachlor-3,3,5,5-tetrafluor-cyclopenten-1, 7,4 % 1,2,4,4-Pentachlor-3,3,5,5-trifluor-cyclopenten-l, 10,1 % 1,2,4,4-Hexachlor-3,3,5,5-difluor-cyclopenten-1 sowie 1,6 % Hexachlorcyclopentadien enthielt.

### Beispiel 6

Nach der Arbeitsweise des Beispiels 1 wurde ein Katalysator aus 295 g Wasser, 25 g 65 %ige HNO₃, 340 g Bi(NO₃)₃.5 H₂O, 50 g Fe(NO₃)₃.9 H₂O und 500 g Calciumoxid hergestellt und bei 270°C mit HF aktiviert.

Molverhältnis ca. Ca:Bi:Fe = 1:0,08:0,02.

Über 130 ml dieses Katalysators wurden während 3 h bei 440°C 149,4 g Hexachlorbutadien, 111 g HF und 49 g Chlor geleitet. Bei der Kondensation der Reaktgionsgase in Eiswasser bildeten sich 138 g einer organischen Phase, die 51,3 % Hexachlorbutadien, 5,6 % Tri-chlor-tri-fluorcyclobuten, 11,3 % Dichlortetrachlorcyclobuten und 18,7 % einer Komponente C₆Cl₅F enthielt.

### Beispiel 7

Aus 25 g 65 Xiger HNO₃, 500 g Wasser, 340 g Bi(NO₃)₃.5 H₂O, 100 g Mn(NO₃)₂.6 H₂O und 270 g MgO wurde gemäß Beispiel 1 ein Katalysator hergestellt und aktiviert.

Molverhältnis Mg:Bi:Mn = 1 : 0,10 : 0,05,

Über 130 ml dieses Katalysators wurden im Laufe von 4 h bei 410°C 79,7 g Hexachlorbutadien, 140 g HF und 7,2 g Chlor geleitet. Bei der Kondensation der Reaktionsgase in Eiswasser bildeten sich 59,8 g einer organischen Phase, die nach GC-Analyse 5,8 % 2,3-Dichlor-1,1,1,4,4,4-hexafluorbuten-2, 11,9 % 1,2-Dichlor-3,3,4,4-tetrafluorcyclobuten-1, 23,1 % 1,2,3-Trichlor-3,4,4-trifluorcyclobuten-1 und 14,7 % Tetrachlor-difluorcyclobutan sowie 13,9 % Hexachlorcyclobutadien enthielten.

### Beispiel 8

Nach Beispiel 1 wurde aus 370 g MgO, 251,6 g Bi(NO₃)₃.5H₂O, 18,5 g 65%iger Salpetersäure und 100 g Wasser ein Katalysator hergestellt und mit HF aktiviert.

Molverhältnis: Mg:Bi = 1:1,32

Über 130 ml des Katalysators wurden im Lauge von S h 99,6 g Hexachlorbutadien, 175 g HF und 9 g Chlor bei 365°C geleitet. Es bildeten sich 71,4 g einer organischen Phase, die 3,7 % 2,3-Dichlor-1,1,1,4,4,4-hexafluorbuten-2, 12,7 % 1,2-Dichlor-3,3,4,4-tetrafluorcyclobuten-1, 27,4 % 1,2,3-Trichlor-3,4,4-trifluorcyclobuten-1, 14,5 % Tetrachlor-difluor-cyclobuten sowie 24,8 % Hexachlorbutadien enthielt.

### Beispiel 9

Über 220 ml des Katalysators nach Beispiel 4 wurden im Laufe von 4 h 81,6 g Hexachlorcyclopentadien, 108 g HF und 10,8 g Chlor bei 415°C geleitet. Bei der Absorption der Reaktionsgase in Wasser bildeten sich 73,6 g einer organischen Phase, die 3,8 % 1,2-Dichlor-3,3,4,4,5,5-hexafluorcyclopent-1-en, 15,6 % 1,2,4-Trichlor-3,3,4,5,5-pentafluorcyclopent-1-en, 33,7 % 1,2,4,4-Tetrachlor-3,3,5,5-tetrafluorcyclopent-1-en, 17,0 % Pentafluor-trifluorcyclopent-1-en, 13 % Di- und Monofluorchlorcyclopent-1-en sowie 6,6 % Hexachlorcyclopentadien enthielt.

## Patentansprüche

1. Verfahren zum Austausch von Halogen, das verschieden von Fluor ist, gegen Fluor in organischen Verbindungen mittels Fluorwasserstoff in Gegenwart eines Katalysators, dadurch gekennzeichnet, daß ein chromfreier Wismut/Erdalkali-Katalysator eingesetzt wird, der 0,005-0,8 gA Wismut pro Mol der eingesetzten Erdalkaliverbindung enthält, gegebenenfalls als Promotoren 0,01 bis 200:1 g-Atome (bezogen auf Wismut) mindestens einer Verbindung von Elementen der VII. und/oder VIII. Nebengruppe der Periodensystems der Elemente (Mendelejew) und/oder der Lanthanide und/oder inerte Zusatzstoffe enthält, man in Gegenwart von 0,1 bis 1,5 Mol Chlor in elementarer Form pro Mol der umzusetzenden organischen Verbindung arbeitet und die Katalysatoren bei 20 bis 500°C mit überschüssigem Fluorwasserstoff vorbehandelt.

2. Verfahren nach Anspruch 1, dadurch gekennzeichnet, daß die organische Verbindung ein perchloriertes geradkettiges oder verzweigtes Alkan, Alken oder Alkadien mit 2-8 C-Atomen oder ein perchloriertes Cycloalken oder Cycloalkadien mit 4-7 C-Atomen ist.

3. Wismut/Erdalkalimetall-Katalysatoren für den Austausch von Halogen, das verschieden von Fluor ist, gegen Fluor in organischen Verbindungen mittels Fluorwasserstoff in Gegenwart von 0,1 bis 1,5 Mol Chlor in elementarer Form pro Mol der umzusetzenden organischen Verbindung, die 0,005-0,8 gA Wismut pro Mol der eingesetzten Erdalkaliverbindung und gegebenenfalls als Promotoren 0,01 bis 200:1 g-Atome (bezogen auf Wismut) mindestens einer Verbindung von Elementen der VII. und/oder VIII. Nebengruppe des Periodensystems der Elemente (Mendelejew) und/oder der Lanthanide und/oder inerte Zusatzstoffe enthalten können und bei 20 bis 500°C mit überschüssigem Fluorwasserstoff vorbehandelt werden.

4. Wismut-Katalysatoren nach Anspruch 3, deren Erdalkaliverbindung aus mindestens einer Verbindung aus der Gruppe der Carbonate, Hydroxide, Oxide und Fluoride der Erdalkalimetalle hervorgegangen ist.

## Claims

1. Process for exchanging halogen other than fluorine for fluorine in organic compounds by means of hydrogen fluoride in the presence of a catalyst, characterized in that a chromium-free bismuth/alkaline earth metal catalyst is used which contains 0.005-0.8 g-atom of bismuth per mol of the alkaline earth metal compound employed, optionally contains, as promoters, 0.01 to 200 g-atoms (based on bismuth) of at least one compound of elements of subgroups VII and/or VIII of the Periodic Table of the Elements (Mendeleev) and/or of the lanthanides and/or inert additives, the operation is carried out in the presence of 0.1 to 1.5 mol of chlorine in elemental form per mol of the organic compound to be reacted and the catalysts are pretreated with excess hydrogen fluoride at 20 to 500°C.

2. Process according to Claim 1, characterized in that the organic compound is a perchlorinated straight-chain or branched alkane, alkene or alkadiene having 2-8 C atoms or a perchlorinated cycloalkene or cycloalkadiene having 4-7 C atoms.

3. Bismuth/alkaline earth metal catalysts for the exchange of halogen other than fluorine for fluorine in organic compounds by means of hydrogen fluoride in the presence of 0.1 to 1.5 mol of chlorine in elemental form per mol of the organic compound to be reacted, which can contain 0.005-0.8 g-atom of bismuth per mol of the alkaline earth metal compound employed, and optionally, as promoters, 0.01 to 200 g-atoms (based on bismuth) of at least one compound of elements of subgroups VII and/or VIII of the Periodic Table of the Elements (Mendeleev) and/or of the lanthanides, and/or inert additives, and are pretreated with excess hydrogen fluoride at 20 to 500°C.

4. Bismuth catalysts according to Claim 3, in which the alkaline earth metal compound originates from at least one compound from the group comprising carbonates, hydroxides, oxides and fluorides of the alkaline earth metals.

## Revendications

1. Procédé pour le remplacement d'un atome d'halogène qui est différent d'un atome de fluor, par un atome de fluor dans des composés organiques au moyen d'acide fluorhydrique en présence d'un catalyseur, caractérisé en ce qu'on met en oeuvre un catalyseur de bismuth/métal alcalino-terreux exempt de chrome qui contient 0,005-0,8 atome-gramme de bismuth par mole du composé de métal alcalino-terreux mis en oeuvre, contenant éventuellement comme promoteurs de 0,01 à 200:1 atomes-gramme (rapportés au bismuth) d'au moins un composé des éléments des groupes secondaires VII et/ou VIII du système périodique des éléments (Mendeleïev) et/ou des lanthanides et/ou des additifs inertes, on travaille en présence de 0,1 à 1,5 mole de chlore sous forme élémentaire par mole du composé organique à transformer et on soumet les catalyseurs à un prétraitement à une température de 20 à 500°C avec de l'acide fluorhydrique en excès.

2. Procédé selon la revendication 1, caractérisé en ce que le composé organique est un alcane, un alcène ou un alcadiène perchloré à chaîne droite ou ramifiée contenant de 2 à 8 atomes de carbone ou encore un cycloalcène ou un cycloalcadiène perchloré contenant de 4 à 7 atomes de carbone.

3. Catalyseurs de bismuth/métal alcalino-terreux pour le remplacement d'un atome d'halogène qui est différent d'un atome de fluor, par un atome de fluor dans des composés organiques au moyen d'acide fluorhydrique en présence de 0,1 à 1,5 mole de chlore sous forme élémentaire par mole du composé organique à transformer, qui peuvent contenir 0,005-0,8 atome-gramme de bismuth par mole du composé de métal alcalino-terreux mis en oeuvre et éventuellement comme promoteurs de 0,01 à 200:1 atomes gramme (rapportés au bismuth) d'au moins un composé des éléments des groupes secondaires VII et/ou VIII du système périodique des éléments (Mendeleïev) et/ou des lanthanides et/ou des additifs inertes, et on les soumet à un prétraitement à une température de 20 à 500°C avec de l'acide fluorhydrique en excès.

4. Catalyseurs de bismuth selon la revendication 3, dont le composé de métal alcalino-terreux dérive d'au moins un composé du groupe des carbonates, des hydroxydes, des oxydes et des fluorures des métaux alcalino-terreux.
